(19) Europäisches Patentamt
European Patent Office
Office européen des brevets

(11) **EP 3 332 710 B1**

(12) **EUROPÄISCHE PATENTSCHRIFT**

(45) Veröffentlichungstag und Bekanntmachung des Hinweises auf die Patenterteilung:
**16.09.2020   Patentblatt 2020/38**

(51) Int Cl.:
*A61B 6/03* (2006.01)      *A61B 6/00* (2006.01)

(21) Anmeldenummer: **16203444.1**

(22) Anmeldetag: **12.12.2016**

(54) **CHARAKTERISIERUNG VON PLAQUE**

CHARACTERISATION OF PLAQUE

CARACTÉRISATION DE PLAQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**13.06.2018   Patentblatt 2018/24**

(73) Patentinhaber: **Siemens Healthcare GmbH**
**91052 Erlangen (DE)**

(72) Erfinder:
• **Flohr, Thomas**
**91486 Uehlfeld (DE)**
• **Schmidt, Bernhard**
**90766 Fürth (DE)**

(56) Entgegenhaltungen:
**JP-A- 2009 178 517      US-A1- 2012 207 270**
**US-A1- 2014 050 378**

• **WILLIAM PAVLICEK ET AL: "Initial use of fast switched dual energy CT for coronary artery disease",** SPIE - INTERNATIONAL SOCIETY FOR OPTICAL ENGINEERING. PROCEEDINGS, Bd. 7622, 4. März 2010 (2010-03-04), Seite 76221V, XP055372522, US ISSN: 0277-786X, DOI: 10.1117/12.844859 ISBN: 978-1-5106-0753-8
• **DANIEL R. OBAID ET AL: "Dual-energy computed tomography imaging to determine atherosclerotic plaque composition: A prospective study with tissue validation",** JOURNAL OF CARDIOVASCULAR COMPUTED TOMOGRAPHY, Bd. 8, Nr. 3, 1. Mai 2014 (2014-05-01), Seiten 230-237, XP055372506, AMSTERDAM, NL ISSN: 1934-5925, DOI: 10.1016/j.jcct.2014.04.007
• **L BOUSSEL ET AL: "Photon counting spectral CT component analysis of coronary artery atherosclerotic plaque samples",** BRITISH JOURNAL OF RADIOLOGY., Bd. 87, Nr. 1040, 1. August 2014 (2014-08-01), Seite 20130798, XP055325294, GB ISSN: 0007-1285, DOI: 10.1259/bjr.20130798

**Beschreibung**

Die Erfindung betrifft ein Verfahren zur Charakterisierung von Plaque, eine Analyseeinrichtung sowie ein Computertomographiesystem.

Gefäßkrankheiten stellen eine wachsende Herausforderung an die Medizin in der immer älter werdenden Bevölkerung dar. Atherosklerose beispielsweise wird durch Plaques in den Gefäßen, insbesondere in den Koronarien gekennzeichnet. Löst sich Plaque von den Gefäßwänden, wird dies als sogenannte Plaque-Ruptur bezeichnet. Wenn eine Plaque-Ruptur in einer Koronarie vorliegt, kann es zum Gefäßverschluss und damit zum Herzinfarkt kommen.

Es gibt Hinweise darauf, dass die Zusammensetzung der Plaques und ihre Morphologie Faktoren sind, die eine Aussage über die Gefährlichkeit der Plaques ermöglichen und zur Vorhersage der Rupturwahrscheinlichkeit dienen können. Lipide Plaques etwa werden als besonders rupturgefährdet eingeschätzt, kalzifizierte Plaques stellen dagegen eher eine stabile Endstufe der koronaren Herzkrankheit dar. Die Zusammensetzung der Plaques kann somit als Indiz für eine folgende Behandlung dienen. Beim Vorliegen besonders gefährlicher Plaques kann z. B. eine aggressive Therapie mit Statinen, beta-Blockern etc. eingeleitet oder das Gefäß mit einem Stent versorgt werden. Bei medikamentöser Therapie sind Nachfolgeuntersuchungen der zu therapierenden Plaques indiziert.

Bisher werden zur Charakterisierung von Plaques meist Verfahren wie beispielsweise intravaskulärer Ultraschall (IVUS) eingesetzt, bei dem eine Ultraschallsonde in das zu untersuchende Gefäß eingeführt wird.

Die Computertomographie (CT) spielt bei der Charakterisierung von Plaques gegenüber diesen Verfahren bis heute nur eine eher untergeordnete Rolle. Dies liegt zum einen daran, dass die Plaquezusammensetzung bislang üblicherweise allein auf Basis von CT-Werten, also Grauwertes (in Hounsfield-Einheiten) der Pixel in einem rekonstruierten Schichtbild bestimmt wird. Dabei werden in der Regel Plaques mit niedrigen CT-Werten im Bereich von Fett als "lipide" charakterisiert, Plaques mit mittleren CT-Werten im Bereich von Weichgewebe als "fibrös", Plaques mit hohen CT-Werten als "kalzifiziert". Eine exakte Klassifizierung eines Plaques ist bei dieser simplen Analyse aber durch einen starken Überlapp der CT-Wert-Bereiche der drei genannten Plaque-Klassen erschwert. Zum anderen hängen die CT-Werte stark von der bei der Aufnahme verwendeten Röhrenspannung ab. Daraus folgt, dass die aus Aufnahmen bei der Standardspannung von 120 kV abgeleiteten CT-Wert-Bereiche für lipide, fibröse und kalzifizierte Plaques sich nicht auf CT-Aufnahmen bei anderen Röhrenspannungen (z. B. 80 kV oder 100 kV) übertragen lassen. Die Röhrenspannungen legen dabei eine maximale Energie der Röntgenstrahlung fest, weshalb mittels unterschiedlicher Röhrenspannungen auch unterschiedliche Röntgenspektren erzeugt werden. Die Röhrenspannung dient daher häufig als Bezeichnung bzw. Charakterisierung für ein entsprechendes Röntgenspektrum. Insgesamt ist aber die alleinige Messung des CT-Wertes zur Charakterisierung der Plaque-Zusammensetzung von limitierter Aussagekraft. William Pavlicek et al., "Initial use of fast switched dual energy CT for coronary artery disease", SPIE Proceedings, Bd. 7622, 4. März 2010 (2010-03-04), Seite 76221V, offenbart ein Verfahren zur Charakterisierung von Plaque mittels Dual-Energy CT, wobei eine Basismaterialzerlegung verwendet wird.

Es ist eine Aufgabe der vorliegenden Erfindung, ein Verfahren zur Charakterisierung von Plaque, eine Analyseeinrichtung sowie ein Computertomographiesystem anzugeben, womit eine verbesserte Charakterisierung von Plaque auf Basis von CT-Daten möglich ist.

Diese Aufgabe wird durch ein Verfahren zur Charakterisierung von Plaque nach Anspruch 1, eine Analyseeinrichtung nach Anspruch 10 sowie ein Computertomographiesystem nach Anspruch 11 gelöst.

Das eingangs genannte Verfahren dient der Charakterisierung von Plaque in einem interessierenden Bereich innerhalb eines Untersuchungsobjekts. Die Charakterisierung erfolgt mittels einer Mehrzahl von Bilddatensätzen, die aus einer Mehrzahl von Projektionsdatensätzen rekonstruiert wurden. Letztere wurden mittels eines CT-Geräts unter Verwendung von jeweils unterschiedlichen Röntgenenergiespektren akquiriert. Das Verfahren weist zumindest folgende Schritte auf: In einem Schritt werden die Bilddatensätze erfasst, die eine Vielzahl von Bildpunkten umfassen. In einem weiteren Schritt werden bildpunktweise spektrale Parameterwerte unter Verwendung von zumindest zwei Bilddatensätzen ermittelt. Auf Basis der spektralen Parameterwerte werden in einem weiteren Schritt bildpunktweise Charakterparameterwerte zur Charakterisierung der Plaques ermittelt.

Als Plaques werden in Rahmen der Erfindung sich chronisch entwickelnde herdförmige Veränderungen an den Zellen der inneren Gefäßwand und/oder der inneren Schichten der mittleren Gefäßwand bezeichnet. Wie eingangs bereits beschrieben, existieren Plaques bzw. Atherosklerose mit unterschiedlichen Zusammensetzungen bzw. Eigenschaften. Daran können die einzelnen Plaquearten unterschieden bzw. charakterisiert werden, wodurch eine Einteilung in unterschiedliche Klassen bzw. Kategorien möglich ist. Die Plaquearten können also beispielsweise als lipide, fibröse oder kalzifizierte Plaques kategorisiert, klassifiziert bzw. charakterisiert werden. Diese Kategorien entsprechen von bezüglich der Werte des spektralen Parameters den namensgebenden Materialien. Ebenso sind aber auch andere - insbesondere auch mehr oder weniger - Kategorien für die Einordnung der jeweiligen Plaquearten möglich.

Dafür wird ein interessierender Bereich untersucht, der vorzugsweise ein Gefäß umfasst, also beispielsweise eine Vene, eine Arterie oder insbesondere eine Koronarie. Dieser Bereich befindet sich im Inneren eines Untersuchungsobjekts, bei dem es sich beispielsweise um einen tierischen, bevorzugt aber um einen menschlichen Patienten handelt.

Die für die Charakterisierung benötigten Daten wurden vor dem eigentlichen Beginn des erfindungsgemäßen Verfahrens mittels eines CT-Gerätes aufgenommen. Dafür wurden zunächst eine Mehrzahl von (also zumindest mehr als ein) Projektionsdatensätzen unter Verwendung von jeweils unterschiedlichen Röntgenenergiespektren akquiriert. Jedem Projektionsdatensatz ist also ein Röntgenenergiespektrum zugeordnet. Er umfasst Werte von lokalen Strahlungsintensitäten der Röntgenstrahlung, die nach einem Durchdringen des Untersuchungsobjekts in einem Detektionselement detektiert werden, d. h. sie werden in einem Pixel eines Detektors des CT-Geräts erfasst. Die Röntgenintensitäten werden dabei aus einer Vielzahl von Winkeln in Bezug zum Untersuchungsobjekt erfasst. Die Röntgenenergiespektren können sich zwischen den Projektionsdatensätzen beispielsweise in ihrer maximalen Energie, ihrer mittleren Energie, ihren charakteristischen Strahlungsmaxima, d. h. allgemein in ihrer spektralen Verteilung, unterscheiden. Aus den Projektionsdatensätzen wird mittels eines bekannten Rekonstruktionsverfahrens, wie beispielsweise der gefilterten Rückprojektion, iterativen Rekonstruktionsverfahren oder dergleichen, eine gleiche Anzahl von Bilddatensätzen rekonstruiert. Als Bilddatensatz wird im Rahmen der Erfindung beispielsweise ein Volumenbild oder ein Schichtbild des Untersuchungsobjekts verstanden. Der Bilddatensatz umfasst dabei eine Vielzahl von Bildpunkten, die üblicherweise bei einem dreidimensionalen Volumendatensatz als Voxel und bei einem zweidimensionalen Schichtbilddatensatz als Pixel bezeichnet werden.

In einem ersten Verfahrensschritt werden die Bilddatensätze erfasst. Das heißt, sie können beispielsweise direkt aus den oben beschriebenen Akquisitionsschritten bzw. Rekonstruktionsschritten übernommen werden oder zum Beispiel in einem Speicher hinterlegt sein und daraus für das erfindungsgemäße Verfahren abgerufen werden. Aus den Bilddatensätzen werden in einem weiteren Schritt spektrale Parameterwerte zu zumindest einem spektralen Parameter bildpunktweise ermittelt. "Bildpunktweise" heißt in diesem Zusammenhang, dass die Werte Pixel für Pixel bzw. Voxel für Voxel ermittelt werden. Die spektralen Parameterwerte stellen somit in jedem Bildpunkt eine Kombination der Bilddaten aus den Bilddatensätzen mit unterschiedlichsten Röntgenenergiespektren da. Dafür können ggf. nach einer Registrierung der Datensätze aufeinander lediglich zwei in den Bilddatensätzen korrespondiere Pixel kombiniert werden. Alternativ können auch Bereiche von Pixeln kombiniert werden, die in einer Umgebung um ein zentrales Pixel herum angeordnet sind, für das der spektrale Parameterwert ermittelt werden soll.

Durch diese Kombination enthalten die spektralen Parameterwerte eine spektrale Information zu dem jeweiligen Bildpunkt. Grundsätzlich können die spektralen Parameterwerte aus einer beliebigen Anzahl von Bilddatensätzen mit unterschiedlichen Spektren ermittelt werden. In einer einfachen und bevorzugten Variante des erfindungsgemäßen Verfahrens werden die spektralen Parameterwerte jedoch aus genau zwei Bilddatensätzen, nämlich einem Niedrigenergiebilddatensatz und einem Hochenergiebilddatensatz ermittelt. Dabei lassen sich nicht nur Werte zu lediglich einem spektralen Parameter bestimmen, vielmehr ist auch eine Ermittlung von Werten zu mehreren unterschiedlichen spektralen Parametern möglich, welche unterschiedliche spektrale Informationen enthalten, wie später noch näher erläutert wird.

Auf Basis der spektralen Parameterwerte werden folgend zur Charakterisierung der Plaques bildpunktweise Charakterparameterwerte ermittelt. Im einfachsten Fall können die Plaques direkt anhand der zu einem spektralen Parameter ermittelten Werte charakterisiert werden. Dazu können beispielsweise Bereiche für spektrale Parameterwerte definiert werden, die der Plaque in dem jeweiligen Bildpunkt eine Klasse bzw. eine Kategorie zuweisen. Es ist jedoch auch möglich, Werte von unterschiedlichen spektralen Parametern zunächst zu kombinieren und dann auf Grundlage dieser Kombination die Charakterisierung der Plaque vorzunehmen, wie später noch näher erläutert wird.

Im Gegensatz zum bekannten Stand der Technik werden die Plaques also nicht allein anhand ihrer Schwächungswerte charakterisiert, sondern auf Basis der spektralen Parameterwerte. Wie oben beschrieben, beinhalten letztere detailliertere Informationen über die Eigenschaften der Plaque. Dadurch wird vorteilhafterweise eine exaktere Charakterisierung der Plaque auf Basis von Daten möglich, die mittels eines CT-Gerätes akquiriert wurden.

Die eingangs genannte Analyseeinrichtung umfasst zur Charakterisierung von Plaque in einem interessierenden Bereich innerhalb eines Untersuchungsobjekts eine Bilddatenschnittstelle, eine Ermittlungseinheit und eine Charakterisierungseinheit. Die Analyseeinrichtung ist dabei so ausgebildet, dass sie die Schritte des erfindungsgemäßen Verfahrens zur Charakterisierung von Plaque ausführt.

Das eingangs genannte Computertomographiesystem umfasst neben einer erfindungsgemäßen Analyseeinrichtung ein CT-Gerät.

Einige wesentliche Komponenten der erfindungsgemäßen Analyseeinrichtung können zum überwiegenden Teil in Form von Softwarekomponenten ausgebildet sein. Dies betrifft insbesondere die Ermittlungseinheit. Grundsätzlich kann diese Komponente aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Computertomographiesysteme und/oder Auswertungscomputer auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Computertomographiesystems ladbar ist, mit Programmabschnitten, um alle Schritte eines der erfindungsgemäßen Verfahren auszuführen, wenn das Programm in dem Computertomographiesystem ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z. B. Hardware-Schlüssel (Dongles etc.) zur Nutzung der Software, umfassen.

Zum Transport zu dem Computertomographiesystem und/oder zur Speicherung an oder in dem Computertomographiesystem kann ein computerlesbares Medium, beispielsweise ein Memorystick, eine Festplatte oder ein sonstiger transportabler oder fest eingebauter Datenträger dienen, auf welchem die von einer Rechnereinheit des Computertomographiesystems einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen oder Beschreibungsteilen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Grundsätzlich können zur Bestimmung einer Materialzusammensetzung auf Basis von CT-Daten beliebig komplexe Verfahren eingesetzt werden. So bietet beispielsweise eine Basismaterialzerlegung eine anspruchsvolle und rechenintensive Methode, möglichst umfassende Materialinformationen zu erhalten. Dieser Detailgrad ist oftmals für eine ausreichende Diagnose nicht erforderlich. Demgegenüber kann es zweckmäßig sein, ein ressourcensparendes, also ein weniger rechenintensives Verfahren zu verwenden.

Die Ermittlung der spektralen Parameterwerte umfasst daher die Ermittlung eines Dual-Energy-Verhältnisses. Das Dual-Energy-Verhältnis bezeichnet dabei in jedem Bildpunkt das Verhältnis vom Schwächungswert eines Niedrigenergiebilddatensatzes zum Schwächungswert eines Hochenergiebilddatensatzes. Typischerweise werden also zwei Bilddatensätze verwendet, wobei der Niedrigenergiebilddatensatz eine niedrigere maximale Röntgenenergie (z. B. 80 KV) und der Hochbildenergiebilddatensatz eine höhere maximale Röntgenenergie (z. B. 140 KV) aufweist.

Ohne den Umfang der Erfindung einzuschränken, werden in der folgenden Beschreibung diese beiden Bilddatensätze stellvertretend für die Mehrzahl von Bilddatensätzen verwendet. Das Dual-Energy-Verhältnis lässt sich also in jedem Bildpunkt durch eine Rechenoperation, nämlich eine Quotientenbildung aus einem entsprechenden Bildpunktwert des Niedrigenergiebilddatensatzes und einem entsprechenden Bildpunktwert des Hochenergiebilddatensatzes berechnen. Nimmt das Dual-Energy-Verhältnis Werte (DE-Ratio) ein, die kleiner sind als eine definierte Schwelle, bevorzugt kleiner eins (DE-Ratio < 1), kann dadurch die Plaque dem entsprechenden Bildpunkt als lipide charakterisiert werden. Bildpunkte mit Werten des Dual-Energy-Verhältnisses in einem Bereich von etwa eins (DE-Ratio ~ 1) sind bevorzugt als fibrös zu klassifizieren, wohingegen Plaque mit Werten des Dual-Energy-Verhältnisses, die größer sind als eins (DE-Ratio > 1), als kalzifiziert einzuordnen ist.

Das Dual-Energy-Verhältnis ist üblicherweise eine konzentrationsunabhängige Größe. Das heißt, es charakterisiert die vorkommenden Materialien unabhängig von ihrer lokalen Dichte. Allerdings kann es bei der Charakterisierung von Plaque einen Vorteil bieten, auch die Materialkonzentration zu berücksichtigen. Daher umfasst die Ermittlung der spektralen Parameterwerte bevorzugt eine Ermittlung eines Dual-Energie-Index. Der Dual-Energie-Index wird für jeden Bildpunkt aus dem Hochenergiebilddatensatz und dem Niedrigenergiebilddatensatz wie folgt berechnet:

$$\frac{BD_{LE}(x) - BD_{HE}(x)}{BD_{LE}(x) + BD_{HE}(x) + \alpha}$$

Dabei bezeichnet $BD_{LE}(x)$ einen Bildpunktwert des Pixels x im Niedrigenergiebilddatensatz und $BD_{HE}(x)$ einen Bildpunktwert des Pixels x im Hochenergiebilddatensatz $BD_{HE}$. Der Parameter $\alpha$ ist ein darstellungsabhängiger Normierungsfaktor. Der Dual-Energie-Index beinhaltet dabei seiner Art nach sowohl Informationen über die atomare Zusammensetzung (Ordnungszahl) des Materials als auch über die Materialkonzentration. Die anschließende Charakterisierung der Plaque kann somit vorteilhafterweise auf Basis des Dual-Energie-Index (DE-Index) vorgenommen werden. Bei Werten des Dual-Energie-Index, die kleiner sind als null (DE-Index < 0, besonders bevorzugt DE-Index < - 0,05), wird die Plaque bevorzugt als lipide charakterisiert. Demgegenüber wird Plaque mit Werten des Dual-Energie-Index, die größer sind als null (DE-Index > 0, besonders bevorzugt DE-Index > 0,05), bevorzugt als kalzifiziert eingeordnet. Plaque mit Werten des Dual-Energie-Index in einem Bereich um null (DE-Index ~ 0, besonders bevorzugt - 0,05 < DE-Index < 0,05) werden bevorzugt als fibrös klassifiziert.

Es ist allerdings auch möglich, den Dual-Energy-Index zusätzlich zum Dual-Energy-Verhältnis für die Charakterisierung von Plaque zu verwenden. Dafür wird besonders bevorzugt aus den beiden spektralen Parametern ein Kombinationsparameter ermittelt, der die Vorteile beider Parameter vereint. So kann beispielsweise der Kombinationsparameter mittels einer gewichteten Addition berechnet werden, die je nach Bedarf die Konzentrationsabhängigkeit oder die Abhängigkeit von der Materialzusammensetzung hervorhebt.

Bei dem erfindungsgemäßen Verfahren werden bevorzugt in einem zusätzlichen Schritt Risikoparameterwerte auf Basis der Charakterparameterwerte ermittelt. Unter Risikoparameterwerten werden dabei Werte für die Wahrscheinlichkeit einer zukünftigen Pathogenese verstanden. Es wird also die Wahrscheinlichkeit für einen möglichen weiteren Verlauf der Krankheit prognostiziert, wie beispielsweise das Risiko für eine weitere Anlagerung von Plaque oder eine Plaque-Ruptur. Die Risikoparameterwerte können grundsätzlich allein auf Basis der Charakterparameterwerte ermittelt werden, es können jedoch weitere Parameter berücksichtigt werden, wie folgend noch näher erläutert wird.

Bevorzugt erfolgt die Ermittlung der Risikoparameterwerte unter Verwendung von morphologischen Daten. Die morphologischen Daten können dabei Werte für ein Plaquevolumen, eine Plaquelänge, eine Position der Plaque innerhalb des Gefäßes oder dergleichen umfassen. Das Plaquevolumen kann dabei das Gesamtvolumen einer Plaque bezeichnen, es kann aber auch die Volumina von einzelnen Plaque-Bereichen beschreiben, welche zuvor charakterisiert worden sind. Die Plaquelänge bezeichnet die Länge der Plaque entlang einer Längsrichtung des Gefäßes bzw. in Richtung des Blutflusses. Aus dem Plaquevolumen und der Plaquelänge lässt sich beispielsweise zudem ein Formparameter der Plaque ermitteln, der ein Verhältnis von der Plaquelänge zu einer Plaquedicke definiert. Daraus können zum Beispiel Rückschlüsse auf die Wahrscheinlichkeit einer Plaque-Ruptur getroffen werden.

Auch die Position der Plaque innerhalb des Gefäßes, also beispielsweise vor Verzweigungen oder dergleichen, kann wesentliche Einflüsse auf die Entwicklung der Plaque haben. Zudem kann das Risiko bzw. die weitere Entwicklung der Plaque durch ein sogenanntes "positives Remodeling" beeinflusst werden. Damit wird eine Ausdehnung des Gefäßes beschrieben, die als Reaktion auf die Plaquebildung erfolgt. Das Gefäß erweitert dabei seinen Querschnitt, um sein ursprüngliches Lumen zu erhalten. Eine derartige Veränderung kann somit wesentlichen Einfluss auf die weitere Entwicklung der Plaque haben und wird daher bevorzugt auch bei der Ermittlung des Risikoparameters berücksichtigt.

Die Ermittlung der Risikoparameterwerte erfolgt vorzugsweise unter Verwendung von Blutflussdaten. Die Blutflussdaten umfassen bevorzugt Daten aus einer vorherigen CT-Perfusionsuntersuchung, Daten aus einer Messung zur fraktionellen Flussreserve (FFR) und/oder Daten zur fraktionellen Flussreserve, die mittels eines CT-Geräts erfasst wurden (CT-FFR). Diese Daten geben Aufschluss über den lokal, d. h. an der Position der Plaque, vorherrschenden Blutfluss und können so vorteilhaft dazu beitragen, die Risikoparameterwerte möglichst exakt zu bestimmen. Mit Hilfe der Blutflussdaten können zur Risikoanalyse beliebig komplexe Berechnungen durchgeführt werden, wie z. B. Finite-Elemente-Simulationen (FEM), Strömungssimulationen oder dergleichen. Dadurch können einerseits beispielsweise die vorherrschenden Scherkräfte ermittelt und zu den Haftkräften der Plaque in Beziehung gesetzt werden, woraus sich wiederum Werte des Risikoparameters ermitteln lassen.

Andererseits können diese Daten auch im Rahmen eines maschinell eingelernten Charakterisierungsprozesses berücksichtigt werden, wie später noch näher beschrieben wird.

Auf Basis von zeitgleich bzw. zeitnah aufgenommenen Daten stellt eine Charakterisierung mittels der Charakterparameterwerte bzw. eine Risikobewertung anhand der Risikoparameterwerte eine Momentaufnahme ohne zeitlichen Kontext dar. Bevorzugt umfasst jedoch die Ermittlung der Risikoparameterwerte eine zeitliche Entwicklung der Charakterparameterwerte, der morphologischen Daten und/oder der Blutflussdaten.

Die zeitliche Entwicklung der Parameterwerte bzw. der Daten wird dabei bevorzugt mittels zwei oder mehr zeitlich beabstandeten Datenakquisitionen erfasst. Um die zeitliche Entwicklung festzustellen, werden die zu den unterschiedlichen Zeitpunkten akquirierten Parameterwerte bzw. Daten verglichen. Der Vergleich kann dabei auf jeder Ebene stattfinden. Das heißt, dass beispielsweise die spektralen Parameterwerte von unterschiedlichen Zeitpunkten auf einer rudimentären Auswertungsebene miteinander verglichen werden können, wobei gegebenenfalls zuvor eine Registrierung der Bilddatensätze aufeinander vorgenommen werden muss.

Alternativ werden die zeitlich beabstandeten Risikoparameterwerte auf einer Ergebnisebene miteinander verglichen. Mit anderen Worten werden Folgeuntersuchungen durchgeführt, mit deren Hilfe die Risikoparameterwerte exakter bestimmt werden können. Das heißt, dass eine genauere Vorhersage für die zukünftige Entwicklung der Plaque getroffen werden kann. Ebenso kann die zeitliche Entwicklung als Indikator für eine Therapieantwort dienen, um somit den Erfolg der Therapie bestätigen bzw. widerlegen zu können. Außerdem kann im Rahmen dieser Folgeuntersuchung überprüft werden, ob bzw. inwieweit die Risikoparameterwerte der vorhergehenden Untersuchungen richtig ermittelt wurden. Die Ergebnisse dieser Überprüfung können vorteilhafterweise als Referenz für ein maschinelles Lernverfahren dienen, welches später noch näher beschrieben wird.

Für ein erfindungsgemäßes Verfahren wurden im Vorfeld bevorzugt Projektionsdatensätze in einem Hochauflösungsmodus akquiriert, insbesondere mittels eines CT-Gerätes mit einem photonenzählenden Detektor. Photonenzählende Detektoren sind beispielsweise als direkt konvertierende Halbleiterdetektoren ausgebildet, die die Röntgenstrahlung energieaufgelöst erfassen. Der Hochauflösungsmodus (HRCT) bezeichnet grundsätzlich Aufnahmemodi, bei denen die Auflösung maximiert wird. Dies kann beispielsweise durch eine Überabtastung (kleiner Pitch, Springfokus am Detektor oder dergleichen) und/oder durch eine Optimierung der Geometrie des CT-Geräts (kleiner Fokus, Reduktion der Apertur des Detektors durch UHR-Kamm oder dergleichen) erfolgen. Dadurch können Unsicherheiten bei der Charakterisierung vermieden werden, wie sie beispielsweise durch das sogenannte "Calcium Blooming" entstehen.

Beim erfindungsgemäßen Verfahren umfasst die Ermittlung der Charakterparameterwerte und/oder die Ermittlung der Risikoparameterwerte bevorzugt ein maschinelles Lernverfahren, besonders bevorzugt auf Basis einer Datenbank von Referenz-Untersuchungsobjekten. Im Rahmen des maschinellen Lernverfahrens werden Kriterien ermittelt, die die Ermittlung der Charakterparameterwerte und/oder die Ermittlung der Risikoparameterwerte zusätzlich verbessern. Dabei handelt es sich insbesondere um komplexe Kriterien, mit deren Hilfe eine Recheneinheit bzw. ein Computer beispielsweise auf Basis eines Algorithmus analysieren kann, wie Plaque anhand der spektral unterschiedlichen Bilddatensätze besser charakterisiert werden kann und wie die Aussagekraft des Risikoparameters weiter erhöht werden kann.

Ein bevorzugtes Lernverfahren umfasst dabei folgende Schritte: Zunächst werden Lerndaten, besonders bevorzugt aus der Datenbank von Referenz-Untersuchungsobjekten, erfasst. Die Lerndaten sind Bilddaten, die mit unterschiedlichen Röntgenenergien aufgenommen wurden. Anhand der Lerndaten werden folgend mit Hilfe des erfindungsgemäßen Verfahrens - ggf. nach einer Bildrekonstruktion aus den Rohdaten - eine Vielzahl von Plaques charakterisiert und dazu besonders bevorzugt auch Risikoparameterwerte ermittelt. Die daraus erhaltenen Ergebnisse werden in einem weiteren Schritt kontrolliert bzw. beurteilt. Die Beurteilung kann dabei durch einen Bediener beispielsweise anhand einer Notenskala erfolgen. Die Beurteilung kann aber auch auf Grundlage von Folgeuntersuchungen vorgenommen werden, wobei die zuvor ermittelten Charakterparameterwerte und/oder die Risikoparameterwerte unter Berücksichtigung der Plaqueentwicklung auf ihre statistische Signifikanz hin überprüft werden.

Bei dem erfindungsgemäßen Verfahren werden bevorzugt in einem zusätzlichen Schritt die Charakterparameterwerte und/oder Risikoparameterwerte lokal zugeordnet ausgegeben, besonders bevorzugt angezeigt. Dabei werden die Parameterwerte ganz besonders bevorzugt als Zahl lokal - d. h. einen örtlich korrespondieren Bildpunkt zugeordnet - ausgeben. Sie können beispielsweise an der Position eines Mauszeigers oder als Mittelwert einer interessierenden Region (ROI) angezeigt werden.

Alternativ oder zusätzlich wird ganz besonders bevorzugt ein Überlagerungsbild ausgegeben, das auf einem Bilddatensatz basiert und in dem die Parameterwerte graphisch codiert dargestellt sind. Das heißt, dass beispielsweise ein Schichtbild aus dem Niedrigenergiebilddatensatz (oder dem Hochenergiebilddatensatz) in Grauwerten dargestellt wird. Zudem werden die Charakterparameterwerte bildpunktweise in unterschiedlichen Abstufungen, z. B. rot, gefärbt visualisiert und die Risikoparameterwerte in unterschiedlichen Abstufungen, z. B. grün, dargestellt. In einer solchen Darstellung ist für einen Bediener leicht zu erkennen, welche Kategorien von Plaque an welchen Positionen im Gefäß angeordnet sind und wie hoch lokal das Risiko für eine Plaque-Ruptur einzuschätzen ist.

Die Ausgabe kann alternativ oder zusätzlich auch in eine direkt oder per Netzwerk verbundene Speichereinrichtung erfolgen, in der sie hinterlegt ist und aus der sie zu einem späteren Zeitpunkt abgerufen werden kann.

Bevorzugt kann das erfindungsgemäße Verfahren auch eine Segmentierung umfassen. Die Segmentierung kann beispielsweise automatisch mittels bekannter Segmentierungsmethoden oder händisch durch einen Benutzer erfolgen. Im Rahmen der Segmentierung werden die Bilddatensätze in Bereiche eingeteilt, in denen sich beispielsweise erstens mit Jod kontrastiertes Blut, zweitens Gefäßwände oder Bindegewebe und drittens Anlagerung von Plaque befindet. Die weiteren Schritte des Verfahrens können dann vorteilhafterweise wir auf jene Segmente angewendet werden, die sich innerhalb der Gefäßwände befinden, aber kein kontrastiertes Blut enthalten.

Vorzugsweise kann der interessierende Bereich (ROI), auf den das erfindungsgemäße Verfahren angewendet wird, zuvor automatisch oder durch einen Benutzer festgelegt werden. Dies ist vorteilhafterweise ressourcensparend, da beispielsweise die erforderlichen Ermittlungsschritte nicht auf den gesamten von den Bilddaten erfassten Bereich, sondern eben nur auf die interessierenden Bereiche angewendet werden müssen.

Bevorzugt ist das CT-Gerät eines erfindungsgemäßen Computertomographiesystems als Multi-Energy-CT-Gerät ausgebildet. Als Multi-Energy-CT-Gerät wird hierbei ein Dual-Source-CT-Gerät, ein CT-Gerät mit kV-Switching (langsam oder schnell), ein CT-Gerät mit Vorfilterung des Röhrenspektrums, ein CT-Gerät mit Multi-Layer-Detektor und/oder ein CT-Gerät mit einem energieselektiven photonenzählenden Detektor oder dergleichen verstanden.

Besonders bevorzugt ist das CT-Gerät zu einer EKGgetriggerten oder einer EKG-gegateten Akquisition der Projektionsdaten ausgebildet. Dadurch werden vorteilhafterweise auch Untersuchungen sich bewegender Gefäße, wie beispielsweise der Koronarien, möglich.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

FIG 1     eine grob schematische Darstellung eines Ausführungs-beispiels eines erfindungsgemäßen Computertomogra-phiesystems,

FIG 2     ein schematisches Blockschaltbild eines Ausführungs-beispiels einer erfindungsgemäßen Analyseeinrichtung,

FIG 3     eine blockschematische Darstellung eines einfachen Ausführungsbeispiels eines erfindungsgemäßen Verfah-rens,

FIG 4     eine blockschematische Darstellung eines weiteren Ausführungsbeispiels eines erfindungsgemäßen Verfah-rens und

FIG 5     vier schematische Schnittansichten durch ein Gefäß eines Patienten, die unterschiedliche Schritte eines erfindungsgemäßen Verfahrens veranschaulichen.

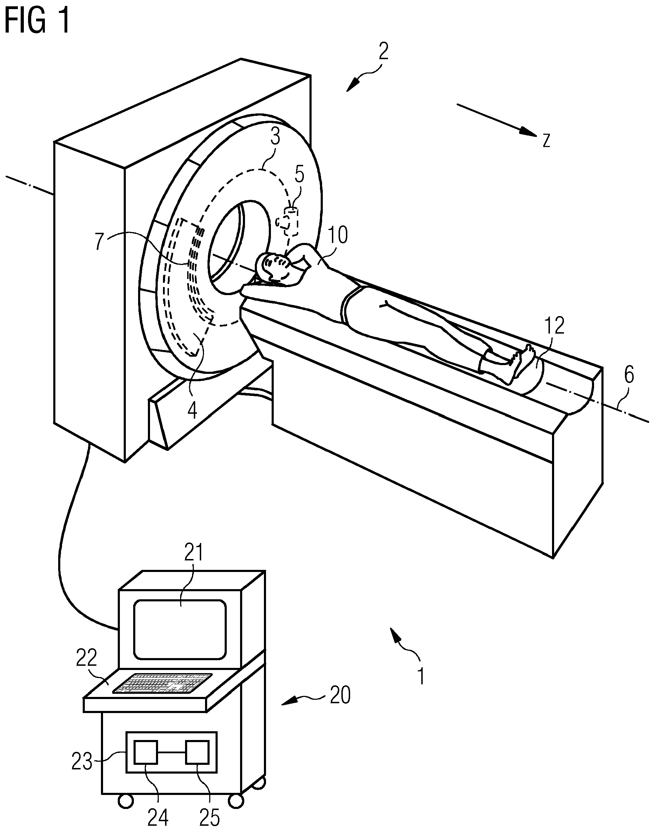

In FIG 1 ist beispielhaft und grob schematisch ein erfindungsgemäßes Computertomographiesystem 1 gezeigt, welches ein Benutzerterminal 20 und ein Computertomographiegerät 2 umfasst. Das Computertomographiesystem 1 ist zur Ausführung des erfindungsgemäßen Verfahrens zur Charakterisierung von Plaque ausgebildet. Das Computertomographiegerät 2 umfasst einen Patiententisch 12 zur Lagerung eines Patienten 10 als Untersuchungsobjekt, welcher entlang einer Systemachse 6 verstellbar ist. Die Systemachse 6 wird im Folgenden auch als z-Achse bezeichnet, die mit dem Patienten 10 in das Messfeld verstellbar ist. Es umfasst ferner eine Gantry 3 mit einer um die Systemachse 6 drehbar gelagerten Quelle-Detektor-Anordnung 4, 5. Die Quelle-Detektor-Anordnung 4, 5 weist eine Röntgenstrahlungsquelle 5 und einen quantenzählenden Detektor 4 auf, die einander gegenüberliegend so ausgerichtet sind, dass im Betrieb eine von dem Fokus der Röntgenstrahlungsquelle 5 ausgehende Röntgenstrahlung auf den Detektor 4 trifft. Die Röntgenquelle ist zum schnellen kV-Switching ausgebildet. Das heißt, sie wechselt während eines Scans ihre Röhrenspannung zwischen 80 kV und 140 kV mit einer Frequenz von beispielsweise 1000 Hz. Der Detektor 4 ist zur ortsaufgelösten Erfassung der Röntgenstrahlung in einzelne Pixel 7 strukturiert, die zu mehreren Detektorzeilen angeordnet sind. Derzeit werden Detektoren 4 eingesetzt, die über insgesamt 64 oder mehr Zeilen verfügen und eine Ortsauflösung im Submillimeterbereich aufweisen. Zu jeder Projektion erzeugt der Detektor 4 einen Satz von Projektionsdaten. Die Projektionsdaten repräsentieren dabei die Schwächungswerte sämtlicher Pixel 7 einer durch den Patienten 10 geschwächten Röntgenstrahlung. Sie werden je nach ihrer Energie in separaten Bins der Pixel 7 des Detektors 4 erfasst.

Ein solches Computertomographiegerät 2 wird bekanntermaßen zur 3D-Bildrekonstruktion eingesetzt. Zur Aufnahme eines Bildes von einem Untersuchungsgebiet (Region of Interest) werden bei Rotation der Quelle-Detektor-Anordnung 4, 5 Projektionsdaten aus einer Vielzahl von unterschiedlichen Projektionsrichtungen erfasst. Im Fall einer Spiralabtastung erfolgt während einer Rotation der Quelle-Detektor-Anordnung 4, 5 beispielsweise gleichzeitig eine kontinuierliche Verstellung des Patiententisches 12 in Richtung der Systemachse 6. Die Röntgenstrahlungsquelle 5 und der Detektor 4 bewegen sich bei dieser Art der Abtastung somit auf einer Helixbahn um den Patienten 10.

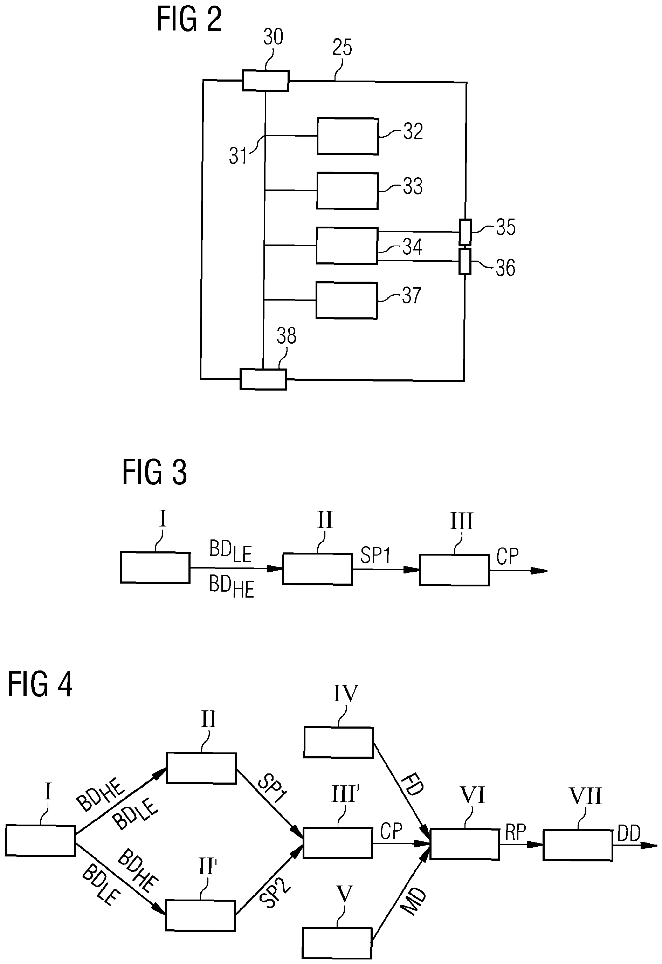

Das Computertomographiesystem 1 umfasst zusätzlich ein Benutzerterminal 20 mit einer Bildrekonstruktionseinrichtung 24 einer Analyseeinrichtung 25, einer Anzeigeeinheit 21, beispielsweise ein Bildschirm, und einer Eingabeeinheit 22, beispielsweise eine Tastatur, zum Erfassen von Benutzereingaben. Die Analyseeinrichtung 25 ist zur Ausführung des erfindungsgemäßen Verfahrens ausgebildet. Die akquirierten Projektionsdaten werden zunächst mit einem bekannten Verfahren rekonstruiert und dann mittels des erfindungsgemäßen Verfahrens zu einem resultierenden überlagerten Bild verrechnet, welches z. B. auf der Anzeigeeinheit 21 darstellbar ist und/oder welches in einem Speicher hinterlegt und/oder an andere Systeme versandt werden kann. Die detaillierte Ausgestaltung der Analyseeinrichtung 25 wird folgend anhand von FIG 2 beschrieben. FIG 3 zeigt beispielhaft ein Blockschaltbild einer erfindungsgemäßen Analyseeinrichtung 25. Sie umfasst eine Bilddatenschnittstelle 30, eine Ermittlungseinheit 32, eine Charakterisierungseinheit 33, eine Bewertungseinheit 34, eine Visualisierungseinheit 37 sowie eine Ausgabeschnittstelle 38, welche mittels eines Busses 31 zur Datenübertragung verbunden sind. Zwischen den Komponenten der Analyseeinrichtung 25 können Daten über den Bus 31 also frei ausgetauscht werden. Die Analyseeinrichtung 25 umfasst ferner eine Morphologiedatenschnittstelle 35 sowie eine Flussdatenschnittstelle 36, welche mit der Bewertungseinheit 34 verbunden sind.

Mittels der Schnittstellen 30, 35, 36, 38 ist die Analyseeinrichtung 25 z. B. mit einem Netzwerk, Speichereinrichtungen und/oder mit anderen Komponenten des CT-Systems 1 wie der Rekonstruktionseinrichtung 14 verbunden. Sie dienen der Datenübertragung von der Analyseeinrichtung 25 zu diesen Komponenten und ggf. umgekehrt.

In FIG 3 ist ein einfaches Ausführungsbeispiel eines erfindungsgemäßen Verfahrens mit drei Schritten blockschematisch dargestellt, welches mithilfe der anhand von FIG 2 beschriebenen Analyseeinrichtung 25 durchgeführt wird. In einem ersten Schritt I werden zwei Bilddatensätze $BD_{HE}$, $BD_{LE}$ mittels der Bilddatenschnittstelle 30 erfasst. Die Bilddatensätze $BD_{LE}$, $BD_{HE}$ wurden mittels des CT-Geräts 2 in einem vorbereitenden, nicht zu dem erfindungsgemäßen Verfahren gehörenden Akquisitionsschritt zunächst als Projektionsdatensätze mit unterschiedlichen Röntgenenergiespektren (80 kV für den Niedrigenergiebilddatensatz $BD_{LE}$ und 140 kV für den Hochenergiebildersatz $BD_{HE}$) detektiert. Folgend wurden sie zu einem Niedrigenergiebilddatensatz $BD_{LE}$ und einem Hochenergiebilddatensatz $BD_{HE}$ rekonstruiert. Gegebenenfalls werden die Bilddatensätze $BD_{LE}$, $BD_{HE}$ aufeinander registriert, um eine einheitliche Ortszuordnung zu ermöglichen.

In einem zweiten Schritt II wird in der Ermittlungseinheit 32 aus den Bilddatensätzen $BD_{LE}$, $BD_{HE}$ bildpunktweise, d. h. aus den jeweiligen Bildpunktwerten Voxel für Voxel bzw. Pixel für Pixel, jeweils ein Dual-Energy-Verhältnis SP1 als spektraler Parameterwert berechnet. Das Dual-Energy-Verhältnis SP1 ergibt sich dabei in jedem Bildpunkt durch den Quotienten $BD_{LE}/BD_{HE}$ aus dem Bildpunktwert des Niedrigenergiebilddatensatzes $BD_{LE}$ und dem korrespondierenden Bildpunktwert des Hochenergiebilddatensatzes $BD_{HE}$.

In einem dritten Schritt III wird in der Charakterisierungseinheit 33 jeder Bildpunkt auf Basis seines berechneten Dual-Energy-Verhältnisses SP1 einer Plaquekategorie zugeordnet, d. h. lipide bei Werten kleiner als eins (<1), fibrös bei Werten von ca. eins (~1) und kalzifiziert bei Werten größer als eins (>1). Die jeweilige Plaquekategorie entspricht dem Charakterparameterwert CP. Sie kann folgend über die Ausgabeschnittstelle 38 an andere Komponenten des CT-Systems 1 weitergeleitet und so beispielsweise mithilfe der Anzeigeeinheit 21 dargestellt werden.

FIG 4 zeigt eine blockschematische Darstellung eines weiteren Ausführungsbeispiels eines erfindungsgemäßen Verfahrens. Die Schritte I und II werden analog zu dem anhand von FIG 3 geschilderten Verfahren durchgeführt. In einem Schritt II' werden ebenfalls in der Ermittlungseinheit 32 auf Basis der Bilddatensätze $BD_{LE}$, $BD_{HE}$ bildpunktweise Werte des Dual-Energy-Index SP2 mittels folgender Formel als Werte eines zweiten spektralen Parameters berechnet:

$$\frac{BD_{80}(x) - BD_{140}(x)}{BD_{80}(x) + BD_{140}(x) + 2000}$$

Dabei bezeichnet $BD_{80}(x)$ einen Bildpunktwert des Pixels x im Niedrigenergiebilddatensatz $BD_{LE}$ und $BD_{140}(x)$ einen Bildpunktwert des Pixels x im Hochenergiebilddatensatz $BD_{HE}$. Der Normierungsfaktor $\alpha$ ist hier auf 2000 festgelegt worden. In einem abgewandelten dritten Schritt III' werden in der Charakterisierungseinheit 33 aus den Werten des Dual-Energy-Verhältnisses SP1 und des Dual-Energy-Index SP2 Charakterparameterwerte CP ermittelt. Dies erfolgt bildpunktweise mittels einer gewichteten Addition, bei der ein Koeffizient zur Gewichtung je nach Bedarf gewählt wird.

In einem vierten Schritt IV werden mithilfe der Flussdatenschnittstelle 36 Flussdaten FD erfasst, die mittels einer CT-FFR-Untersuchung im Vorfeld aufgenommen wurden. Die Flussdaten FD umfassen dabei beispielsweise ein vollständiges Strömungsprofil innerhalb des Gefäßes oder eine Druckdifferenz, die zwischen Positionen vor bzw. hinter der Plaque herrscht.

Außerdem werden in einem fünften Schritt V morphologische Daten MD mittels der Morphologiedatenschnittstelle 35 erfasst, die zuvor beispielsweise aus einer Strukturanalyse der Bilddatensätze $BD_{LE}$, $BD_{HE}$ gewonnen wurden. Die morphologischen Daten MD umfassen dabei beispielsweise eine Länge, ein Volumen und/oder einen Formparameter der Plaque. Gegebenenfalls wird mittels der morphologischen Daten MD auch ein "positives Remodeling", also ein reaktiver gewerblicher Umbauvorgang auf die erhöhte physiologische bzw. pathologische Belastung, des Gefäßes erfasst.

In einem sechsten Schritt VI werden bildpunktweise Risikoparameterwerte RP in der Bewertungseinheit 34 ermittelt. Dafür kann beispielsweise eine Simulation durchgeführt werden. Darin fließen zum einen beispielsweise die auf die Plaque wirkende Scherkräfte, die aus in den Flussdaten FD umfassten lokalen Strömungen resultieren ein. Zum anderen werden darin die Form der Plaque auf Basis der morphologischen Daten MD und deren Zusammensetzung auf Basis der Charakterparameterwerte CP berücksichtigt. Alternativ können die Risikoparameterwerte RP auch anhand von komplexen Kriterien ermittelt werden, die zuvor im Rahmen eines maschinellen Lernverfahrens festgelegt wurden.

In einem siebten Schritt VII wird in der Visualisierungseinheit 37 eine Darstellung, d.h. ein auszugebendes Bild, für die Anzeige auf der Anzeigeeinheit 21 erstellt. In die Darstellung können grundsätzlich alle bisher verwendeten Daten FD, MD bzw. Werte SP1, SP2, CP, RP mit einfließen, sodass von einem Benutzer ausgewählt werden kann, welche Elemente dargestellt werden sollen. Der Darstellung kann beispielsweise einer der Bilddatensätze $BD_{LE}$, $BD_{HE}$ zu Grunde gelegt, d.h. als Grauwertdarstellung im Hintergrund verwendet, werden. Die Flussdaten FD können beispielweise mittels Vektoren als Vektorfeld symbolisch dargestellt werden. Die Parameterwerte können beispielsweise farblich (z. B. die Risikoparameterwerte RP in Rot und die Charakterparameterwerte CP in Grün) in Abstufungen, die ihrem Betrag sprechen, als Überlagerung zum Hintergrund eingeblendet werden. Diese graphisch kodierte Darstellung kann interaktiv von einem Benutzer über eine Eingabe mittels der Eingabeeinheit 22 angepasst werden und wird schließlich als Darstellungsdaten DD an die Anzeigeeinheit 21 übertragen und auf dieser angezeigt.

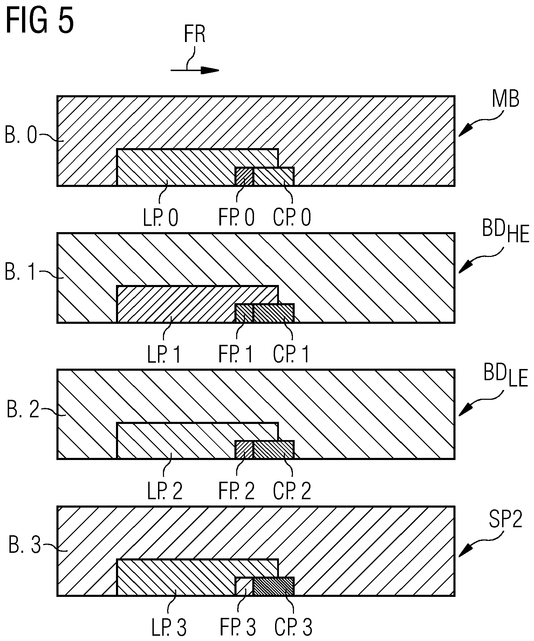

FIG 5 zeigt vier schematische Schnittansichten durch ein Gefäß eines Patienten, die unterschiedliche Schritte eines erfindungsgemäßen Verfahrens veranschaulichen. Das dargestellte Gefäß wird von Blut in einer Flussrichtung FR durchströmt.

In einem abstrahierten Materialbild MB, das die tatsächlich im Gefäß vorherrschende Materialanordnung darstellt, ist das Gefäß zu einem Großteil mit Blut B.0 gefüllt. An einem Rand des Gefäßes haben sich an einer Gefäßwand unterschiedliche Plaquearten LP.0, FP.0 und CP.0 angelagert. In dem Materialbild MB sind lipide Plaque LP.0, fibröse Plaque FP.0 und kalzifizierte Plaque CP.0 deutlich zu unterscheiden.

Die Ansichten $BD_{LE}$, $BD_{HE}$ stellen Schichtbilder aus dem Niedrigenergiebilddatensatz $BD_{LE}$ und dem Hochenergiebilddatensatz $BD_{HE}$ dar. Sämtliche Komponenten der Materialanordnung B.1, LP.1, FP.1 und CP.1 sowie B.2, LP.2, FP.2 und CP.2 sind in Grauwerten wiedergebegeben, was eine Charakterisierung der Plaque erschwert. Insbesondere die fibröse Plaque FP.1 und die kalzifizierte Plaque CP.1 sind in dem Hochenergiebilddatensatz $BD_{HE}$ kaum zu unterscheiden.

Demgegenüber sind in der erfindungsgemäßen Darstellung, die auf dem Dual-Energy-Index SP2 beruht, alle Komponenten B.3, LP.3, FP.3 und CP.3 deutlich zu unterscheiden. Insbesondere können die unterschiedlichen Arten der Plaqueanlagerungen LP.3, FP.3 und CP.3 hier aufgrund der prägnanten Farbdarstellung klar ersichtlich in lipide Plaque LP.3, fibröse Plaque FP.3 und kalzifizierte Plaque CP.3 unterteilt werden, was eine Diagnose und/oder eine Prognose bzw. eine Risikoabschätzung deutlich erleichtert.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit", "Einrichtung", "Gerät" und "System" nicht aus, dass die betreffende Komponente aus mehreren zusammenwirkenden Teilkomponenten besteht, die gegebenenfalls auch räumlich verteilt sein können. Umgekehrt können allerdings beispielsweise die Schnittstellen der Analyseeinrichtung auch in einer gemeinsamen Schnittstelle zusammengefasst sein.

**Patentansprüche**

1. Verfahren zur Charakterisierung von Plaque (LP, FP, CP) in einem interessierenden Bereich innerhalb eines Untersuchungsobjekts (10) mittels einer Mehrzahl von Bilddatensätzen ($BD_{LE}$, $BD_{HE}$), die aus einer Mehrzahl von Projektionsdatensätzen rekonstruiert wurden, welche mittels eines CT-Gerätes (2) unter Verwendung von jeweils unterschiedlichen Röntgenenergiespektren akquiriert wurden, mit zumindest folgenden Schritten:

   - Erfassen (I) der Bilddatensätze ($BD_{LE}$, $BD_{HE}$), die eine Vielzahl von Bildpunkten umfassen,
   - bildpunktweise Ermittlung (II, II') von spektralen Parameterwerten (SP1, SP2), umfassend eine Ermittlung (II) eines Dual-Energy-Verhältnisses, unter Verwendung von zumindest zwei Bilddatensätzen ($BD_{LE}$, $BD_{HE}$),
   - bildpunktweise Ermittlung (III) von Charakterparameterwerten (CP) zur Charakterisierung der Plaques (LP, FP, CP) auf Basis der spektralen Parameterwerte (SP1, SP2).

2. Verfahren nach Anspruch 1, wobei die Ermittlung der spektralen Parameterwerte (SP2) eine Ermittlung (II') eines Dual-Energy-Index umfasst.

3. Verfahren nach einem der vorstehenden Ansprüche, wobei zusätzlich Risikoparameterwerte (RP) auf Basis der Charakterparameterwerte (CP) ermittelt werden.

4. Verfahren nach Anspruch 3, wobei die Ermittlung (VI) der Risikoparameterwerte (RP) unter Verwendung von morphologischen Daten (MD) erfolgt.

5. Verfahren nach Anspruch 3 oder 4, wobei die Ermittlung (VI) der Risikoparameterwerte (RP) unter Verwendung von Blutflussdaten (FD) erfolgt.

6. Verfahren nach einem der Ansprüche 3 bis 5, wobei die Ermittlung (VI) der Risikoparameterwerte (RP) eine zeitliche Entwicklung der Charakterparameterwerte (CP), der morphologischen Daten (MD) und/oder der Blutflussdaten (FD) umfasst.

7. Verfahren nach einem der vorstehenden Ansprüche, wobei die Projektionsdatensätze, insbesondere mittels eines CT-Gerätes (2) mit photonenzählendem Detektor (4), in einem Hochauflösungsmodus akquiriert wurden.

8. Verfahren nach einem der vorstehenden Ansprüche, wobei die Ermittlung (III) der Charakterparameterwerte (CP) und/oder die Ermittlung (VI) der Risikoparameterwerte (RP) ein maschinelles Lernverfahren umfasst, vorzugsweise auf Basis einer Datenbank von Referenz-Untersuchungsobjekten.

9. Verfahren nach einem der vorstehenden Ansprüche, zusätzlich umfassend eine lokal zugeordnete Ausgabe (VII) der Charakterparameterwerte (CP) und/oder der Risikoparameterwerte (RP), wobei die Parameterwerte (CP, RP) als Zahl lokal zugeordnet ausgegeben werden und/oder in einem Überlagerungsbild, das auf einem der Bilddatensätze basiert, graphisch codiert ausgegeben werden.

10. Analyseeinrichtung (25) zur Charakterisierung von Plaque in einem interessierenden Bereich innerhalb eines Untersuchungsobjekts (10) mit

- einer Bilddatenschnittstelle (30) zum Erfassen von einer Mehrzahl von Bilddatensätzen (BD$_{LE}$, BD$_{HE}$), die aus einer Mehrzahl von Projektionsdatensätzen rekonstruiert wurden, welche mittels eines CT-Gerätes (2) unter Verwendung von jeweils unterschiedlichen Röntgenenergiespektren akquiriert wurden,
- einer Ermittlungseinheit (32) zur bildpunktweisen Ermittlung (II, II') von spektralen Parameterwerten (SP1, SP2), umfassend eine Ermittlung (II) eines Dual-Energy-Verhältnisses, unter Verwendung von zumindest zwei Bilddatensätzen (BD$_{LE}$, BD$_{HE}$)und
- einer Charakterisierungseinheit (33) zur bildpunktweisen Ermittlung (III) von Charakterparameterwerten (CP) zur Charakterisierung der Plaques (LP, FP, CP) auf Basis der spektralen Parameterwerte (SP1, SP2).

11. Computertomographiesystem (1) mit einem CT-Gerät (2) und einer Analyseeinrichtung (25) nach Anspruch 10.

12. Computertomographiesystem nach Anspruch 11, wobei das CT-Gerät (2) als Multi-Energy-CT-Gerät (2) ausgebildet ist.

13. Computerprogrammprodukt mit einem Computerprogramm, welches direkt in eine Speichereinrichtung einer Analyseeinrichtung (25) ladbar ist, mit Programmabschnitten, um alle Schritte eines Verfahrens nach einem der Ansprüche 1 bis 9 auszuführen, wenn das Computerprogramm in der Analyseeinrichtung (25) ausgeführt wird.

14. Computerlesbares Medium, auf welchem von einer Rechnereinheit einlesbare und ausführbare Programmabschnitte gespeichert sind, um alle Schritte eines Verfahrens nach einem der Ansprüche 1 bis 9 auszuführen, wenn die Programmabschnitte von der Rechnereinheit ausgeführt werden.


**Claims**

1. Method for the characterisation of plaque (LP, FP, CP) in a region of interest inside an examination subject (10) by means of a plurality of image data sets (BD$_{LE}$, BD$_{HE}$), which have been reconstructed from a plurality of projection data sets, which sets were acquired by means of a CT device (2) using different X-ray energy spectra in each case, comprising at least the following steps:

- acquiring (I) the image data sets (BD$_{LE}$, BD$_{HE}$),

which comprise a plurality of pixels,
- acquisition on a pixel by pixel basis (II, II') of spectral parameter values (SP1, SP2), comprising an acquisition (II) of a dual energy ratio, using at least two image data sets (BD$_{LE}$, BD$_{HE}$),
- acquisition on a pixel by pixel basis (III) of character parameter values (CP) to characterise the plaques (LP, FP, CP) based on the spectral parameter values (SP1, SP2).

2. Method according to claim 1, wherein the acquisition of the spectral parameter values (SP2) comprises acquisition (II') of a dual energy index.

3. Method according to one of the preceding claims, wherein in addition risk parameter values (RP) are determined from the character parameter values (CP).

4. Method according to claim 3, wherein the acquisition (VI) of the risk parameter values (RP) is achieved using morphological data (MD).

5. Method according to claim 3 or claim 4, wherein the acquisition (VI) of the risk parameter values (RP) is achieved using blood flow data (FD).

6. Method according to one of claims 3 to 5, wherein the acquisition (VI) of the risk parameter values (RP) is a chronological evolution of the character parameter values (CP), the morphological data (MD) and/or the blood flow data (FD).

7. Method according to one of the preceding claims, wherein the projection data sets are acquired in particular using a CT device (2) with a photon-counting detector (4), in a high-resolution mode.

8. Method according to one of the preceding claims, wherein the acquisition (III) of the character parameter values (CP) and/or the acquisition (VI) of the risk parameter values (RP) comprises a machine learning process, preferably based on a database of reference examination subjects.

9. Method according to one of the preceding claims, additionally comprising a locally assigned output (VII) of the character parameter values (CP) and/or of the risk parameter values (RP), wherein the parameter values (CP, RP) are output as a figure that is locally assigned and/or output in graphically encoded form in a superimposed image that is based on one of the image data sets.

10. Analytical device (25) for the characterisation of plaque in a region of interest inside an examination subject (10) comprising

- an image data interface (30) for acquiring a plurality of image data sets ($BD_{LE}$, $BD_{HE}$), which have been reconstructed from a plurality of projection data sets, which sets were acquired by means of a CT device (2) using X-ray energy spectra that differ in each case,
- an acquisition unit (32) for acquisition (II, II') on a pixel by pixel basis of spectral parameter values (SP1, SP2), comprising an acquisition (II) of a dual energy ratio, using at least two image data sets ($BD_{LE}$, $BD_{HE}$) and
- a characterisation unit (33) for acquisition on a pixel by pixel basis (III) of character parameter values (CP) for the characterisation of plaques (LP, FP, CP) from spectral parameter values (SP1, SP2).

**11.** Computed tomography system (1) comprising a CT device (2) and an analytical device (25) according to claim 10.

**12.** Computed tomography system according to claim 11, wherein the CT device (2) is designed as a multi-energy CT device (2).

**13.** Computer program product with a computer program that can be loaded directly into a memory unit of an analytical device (25), with program segments to carry out all the steps in a method according to one of claims 1 to 9, when the computer program is running in the analytical device (25).

**14.** Computer-readable medium, on which program segments that are readable and executable by a computation unit are stored in order to carry out all the steps of a method according to one of claims 1 to 9 when the program segments are run by the computation unit.

**Revendications**

**1.** Procédé de caractérisation de plaques (LP, FP, CP) dans une région intéressante au sein d'un objet (10) à examiner, au moyen d'une pluralité d'ensembles ($BD_{LE}$, $BD_{HE}$) de données d'image, qui ont été reconstruites à partir d'une pluralité d'ensembles de données de projection, lesquels ont été acquis au moyen d'un appareil CT (2), en utilisant des spectres d'énergie de rayons X respectivement différents, comprenant au moins les stades suivants :

- relevé (I) des ensembles ($BD_{LE}$, $BD_{HE}$) de données d'image, qui comprennent une pluralité de points d'image,
- détermination (II, II'), point d'image par point d'image, de valeurs (SP1, SP2) de paramètres spectraux, comprenant une détermination (II) d'un rapport d'énergie dual, en utilisant au moins deux ensembles ($BD_{LE}$, $BD_{HE}$) de données d'image,
- détermination (III), point d'image par point d'image, de valeurs (CP) de paramètres de caractère pour la caractérisation des plaques (LP, FP, CP) sur la base des valeurs (SP1, SP2) de paramètres spectraux.

**2.** Procédé suivant la revendication 1, dans lequel la détermination des valeurs (SP2) de paramètres spectraux comprend une détermination (II') d'un indice d'énergie dual.

**3.** Procédé suivant l'une des revendications précédentes, dans lequel on détermine des valeurs (RP) supplémentaires de paramètre de risque sur la base des valeurs (CP) de paramètre de caractère.

**4.** Procédé suivant la revendication 3, dans lequel la détermination (VI) des valeurs (RP) de paramètre de risque s'effectue en utilisant des données (MD) morphologiques.

**5.** Procédé suivant la revendication 3 ou 4, dans lequel la détermination (VI) des valeurs (RP) de paramètre de risque s'effectue en utilisant des données (FD) de flux sanguin.

**6.** Procédé suivant l'une des revendications 3 à 5, dans lequel la détermination (VI) des valeurs (RP) de paramètre de risque comprend un développement dans le temps des valeurs (CP) de paramètre de caractère, des données (MD) morphologiques et/ou des données (FD) de flux sanguin.

**7.** Procédé suivant l'une des revendications précédentes, dans lequel on a acquis les ensembles de données de projection, notamment au moyen d'un appareil CT (2) ayant un détecteur (4) comptant les photons, dans un mode de haute résolution.

**8.** Procédé suivant l'une des revendications précédentes, dans lequel la détermination (III) des valeurs (CP) de paramètre de caractère et/ou la détermination (VI) des valeurs (RP) de paramètre de risque comprend un procédé d'apprentissage automatique, de préférence sur la base d'une base de données d'objets à examiner de référence.

**9.** Procédé suivant l'une des revendications précédentes, comprenant, en outre, une sortie (VII) associée localement des valeurs (CP) de paramètre de caractère et/ou des valeurs (RP) de paramètre de risque, les valeurs (CP, RP) de paramètre étant sorties en étant associées localement sous la forme d'un nombre et/ou étant sorties en étant codées graphiquement dans une image de superposition, qui repose sur l'un des ensembles de données d'image.

10. Dispositif (25) d'analyse pour la caractérisation de plaque dans une région intéressante au sein d'un objet (10) à examiner, comprenant

    - une interface (30) de données d'image pour le relevé d'une pluralité d'ensembles ($BD_{LE}$, $BD_{HE}$) de données d'image, qui ont été reconstruits à partir d'une pluralité d'ensembles de données de projection, lesquels ont été acquis au moyen d'un appareil CT (2), en utilisant des spectres d'énergie à rayons X différents respectivement,
    - une unité (32) de détermination (II, II'), point d'image par point d'image, de valeurs (SP1, SP2) de paramètres spectraux, comprenant une détermination (II) d'un rapport d'énergie dual, en utilisant au moins deux ensembles ($BD_{LE}$, $BD_{HE}$) de données d'image et
    - une unité (33) de caractérisation pour la détermination (III), point d'image par point d'image, de valeurs (CP) de paramètres de caractère, pour la caractérisation des plaques (LP, FP, CP) sur la base des valeurs (SP1, SP2) de paramètres spectraux.

11. Système (1) de tomodensitométrie par ordinateur, comprenant un appareil CT (2) et un dispositif (25) d'analyse suivant la revendication 10.

12. Système de tomodensitométrie par ordinateur suivant la revendication 11, dans lequel l'appareil CT (2) est constitué sous la forme d'un appareil (2) CT à énergie multiple.

13. Produit de programme d'ordinateur ayant un programme d'ordinateur, qui peut être chargé directement dans un dispositif de mémoire d'un dispositif (25) d'analyse ayant des parties de programme pour effectuer tous les stades d'un procédé suivant l'une des revendications 1 à 9, lorsque le programme d'ordinateur est réalisé dans le dispositif (25) d'analyse.

14. Support déchiffrable par ordinateur, sur lequel des parties de programme, pouvant être lues et réalisées par une unité informatique, sont mémorisées, afin d'effectuer tous les stades d'un procédé suivant l'une des revendications 1 à 9, lorsque les parties de programme sont réalisées par l'unité informatique.

FIG 1

# FIG 2

# FIG 3

# FIG 4

FIG 5

FR

MB

B. 0

LP. 0    FP. 0    CP. 0

BD$_{HE}$

B. 1

LP. 1    FP. 1    CP. 1

BD$_{LE}$

B. 2

LP. 2    FP. 2    CP. 2

SP2

B. 3

LP. 3    FP. 3    CP. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **WILLIAM PAVLICEK et al.** Initial use of fast switched dual energy CT for coronary artery disease. *SPIE Proceedings,* 04. Marz 2010, vol. 7622, 76221V